# EUROPEAN PATENT APPLICATION

(11) **EP 2 901 955 A1**
(43) Date of publication of application: **05.08.2015**
(21) Application number: 14179962.7
(22) Date of filing: 06.08.2014
(51) Int. Cl.: A61B 18/20, A61N 5/06

(54) **Portable hair removal apparatus having replaceable laser light beam and cooling cartridges**

(30) Priority: 29.01.2014 KR 20140011461; 04.02.2014 KR 20140012752
(71) Applicant: HABALAN Med & Beauty Co., Ltd., Geumcheon-gu, Seoul 153-704 (KR)
(72) Inventor: Kim, Sang Du, 153-704 Seoul (KR)
(74) Representative: Betten & Resch

(57) **Abstract**

Disclosed is a portable hair removal apparatus using a laser light beam that can be used for various skin beauty treatments such as hair removal, skin massage, skin whitening, and cuticle removal. The portable hair removal apparatus includes a main body with a mounting portion, a laser light beam cartridge which is mounted to or removed from the mounting portion and which is equipped with a laser light beam outputting portion, and a cooling cartridge which is mounted to and removed from the mounting portion and is equipped with a contact-type heat absorbing portion.

## Description

### BACKGROUND OF THE INVENTION

### Field of the Invention

The present invention relates to a portable hair removal apparatus having a replaceable laser light beam cartridge and a replaceable cooling cartridge. More particularly, the present invention relates to a laser light beam emitting apparatus, a hair removal apparatus, used for skin beauty treatments using a laser light beam. The hair removal apparatus is portable and easy to use. Furthermore, the hair removal apparatus can calm down skin which is irritated by heat generated during hair removal treatment which uses a laser light beam, thereby preventing damage to the skin. Moreover, the hair removal apparatus can be used as a multi-purpose skin beauty treatment apparatus for skin massage, skin whitening, cuticle removal, etc. as well as for hair removal.

### Description of the Related Art

Generally, a hair removal apparatus means an apparatus for removing body hair. Conventional hair removal apparatuses are categorized into a razor type that removes body hair using a razor blade and a thresher type that plucks out body hair with force to remove the hair root.

Most of the conventional hair removal apparatuses remove body hair using physical force. The razor type has a problem that it cannot remove hair roots, so that a user cannot avoid periodic removal of body hair. The thresher type has problems of giving pain to a user during hair removal, causing damage to the skin, and expanding hair follicles.

As a conventional art to solve those problems, Korean Patent No. 10-0915371 (registered as of August 27, 2009 and titled "Heat Cutting Type Hair Remove Apparatus) discloses a hair removal apparatus (hereinafter, referred to as Patent Document 1).

Patent Document 1 relates to a hair removal apparatus that removes body hairs while the body hairs are passing between the teeth of a comb of a hair removal portion thereof using heat rays.

Patent Document 1 has the following problems: heat rays come in direct contact with the skin, causing damage to the skin; hair roots are left (i.e. permanent hair removal is impossible) after hairs are removed because the hair removal apparatus removes by burning only the exposed hair part; and it is inconvenient and unsafe to use the hair removal apparatus because body hairs are burned during hair removal.

In order to solve this problem, Korean Utility Model Registration No. 20-0360150 (registered as of August 17, 2004 and titled "Skin Therapy Equipment) discloses a hair removal apparatus that uses a wavelength of light to remove body hair (hereinafter referred to as Patent Document 2).

Patent Document 2 removes body hairs using a hair removal hand piece that emits a laser light beam having a wavelength of 560 nm to 1100 nm. The equipment includes a main body equipped with an operation part for driving the hand piece. Accordingly, the equipment is not portable so that users usually use it by visiting medical care centers. That is, it is difficult for users to easily use the equipment.

Patent Document 2 also has a problem owing to the fact that heat in the skin is not promptly dissipated when the skin is heated by the laser light beam used for hair removal. This residual heat may cause severe irritation to the skin. Accordingly, when an appropriate skin relaxing treatment is not performed, the skin may be damaged.

There is another conventional art, Korean Patent No. 10-0801376 (registered as of January 29, 2008 and titled "Portable Hair Removing Apparatus Using Light Pulse") (hereinafter, referred to as Patent Document 3).

Patent Document 3 removes body hairs using a flash lamp, which is installed in a hollow portion of a body, to emit light pulses. In this case, the body has a chamber between an inner wall and an outer wall that define the hollow portion and the chamber is filled with cooling water. In this way, heat generated during hair removal is eliminated.

Since Patent Document 3 cools down the skin using the cooling water, the resulting cooling effect using the cooling water stored in the portable hair removal apparatus is counterproductive in terms of effective hair removal. Furthermore, when the portable hair removal apparatus is used for a long period of time, the temperature of the cooling water increases, and the cooling effect is deteriorated.

Since Patent Document 3 has a water-cooling-type cooling structure, it is difficult for the hair removal apparatus to have a waterproof structure. Moreover, since hair removal is performed using electric or electronic parts, the hair removal apparatus needs to secure a tight waterproof structure to prevent the electric or electronic parts from malfunctioning or to prevent safety issues such as electric shocks. Accordingly, it is difficult to manufacture the hair removal apparatus. Furthermore, since it is difficult to effectively dissipate heat absorbed by the cooling water, the cooling effect of the hair removal apparatus is limited.

Laser light beams can be used to cause various effects according to their frequencies. However, Patent Document 2 and Patent Document 3 use a laser light beam only for the purpose of hair removal. Accordingly their use is limited. That is, Patent Document 2 and Patent Document 3 are not versatile and have limited usefulness.

The information disclosed in the Background of the Invention section is only for the enhancement of understanding of the background of the invention, and should not be taken as an acknowledgment or as any form of suggestion that this information forms a prior art that would already be known to a person skilled in the art.

### [Related Art Documents]

Patent Document 1: Korean Patent No. 10-0915371;
Patent Document 2: Korean Utility Model Registration No. 20-0360150; and
Patent Document 3: Korean Patent No. 10-0801376.

### SUMMARY OF THE INVENTION

Accordingly, the present invention has been made keeping in mind the above problems occurring in the related art, and the present invention is intended to propose a portable hair removal apparatus having not only a replaceable laser light beam cartridge which has a laser light beam outputting portion used for hair removal and skin massage, but also a replaceable cooling cartridge which has a contact-type heat absorbing portion having an excellent heat dissipating structure which absorbs residual heat in skin through direct contact with the skin and dissipates the absorbed heat, thereby easily and promptly dissipating heat generated during cosmetic therapy such as skin beauty treatments and reducing skin irritation and damage.

Another object of the present invention is to provide a portable hair removal apparatus in which a laser light beam cartridge can be replaced with a cooling cartridge or vice versa when it is used. This improves usability of the laser light beam cartridge and the cooling cartridge and portability of the portable hair removal apparatus.

A further object of the present invention is to provide a multi-purpose laser light beam emitting apparatus used for various skin beauty treatments such as hair removal, skin massage, skin whitening, and cuticle removal by controlling functions of a laser light beam cartridge and a cooling cartridge.

Yet another object of the present invention is to provide a portable hair removal apparatus equipped with a cooling fan installed on a mounting portion of a body, the cooling fan improving the cooling effect of a replaceable cooling cartridge, dissipating heat generated during hair removal which is performed using a laser light beam cartridge in order to alleviate skin irritation, and enabling the portable hair removal apparatus to be used for a long period of time.

Yet further object of the present invention is to provide a portable hair removal apparatus in which a laser light beam cartridge is equipped with a touch sensor which enables the laser light beam cartridge to emit a laser light beam having a user-specified wavelength band and prevents the laser light beam cartridge from erroneously emitting a laser light beam when a user does not want to emit a laser light beam.

Yet a further object of the present invention is to provide a portable hair removal apparatus in which a mounting portion of a body is equipped with an air circulation means which blows in outside air and circulates the outside air through the mounting portion to maximize the cooling effect of a cooling fan.

In order to achieve the objects of the present invention, one aspect of the present invention provides a portable hair removal apparatus including: a main body with a mounting portion; a laser light beam cartridge which is mounted to or removed from the mounting portion and which is equipped with a laser light beam outputting portion; and a cooling cartridge which is mounted to and removed from the mounting portion and is equipped with a contact-type heat absorbing portion.

The mounting portion may be equipped with a cooling fan.

The laser light beam outputting portion of the laser light beam cartridge may be equipped with a touch sensor.

The mounting portion may be further equipped with an air circulation means that ventilates the inside of the mounting portion with outside air.

The portable hair removal apparatus according to the present invention includes a cooling cartridge equipped with a contact-type heat absorbing portion having a heat dissipating structure with an excellent heat dissipating effect in addition to a laser light beam cartridge which emits a laser light beam for skin beauty treatments such as hair removal and skin message. Accordingly, the portable hair removal apparatus can eliminate heat residing in the skin by using the cooling cartridge, thereby preventing skin damage attributable to heat. Furthermore, the portable hair removal apparatus does not have a forceful cooling mechanism such as a cooling-water-type cooling mechanism but has a natural cooling mechanism that naturally cools down the skin by absorbing the heat through direct contact between the cooling cartridge and the skin. Accordingly, the portable hair removal apparatus has an advantage of preventing skin irritation attributable to rapid cooling.

According to the present invention, since the laser light beam cartridge and the cooling cartridge are selectively mounted within a single body of the portable hair removal apparatus by replacing one with the other, the cartridges can be conveniently used, and the structure of the body can be simplified. In addition, since the portable hair removal apparatus has a structure resulting in a good heat dissipation effect, the portable hair removal apparatus has good cooling effect and is easy to carry and use.

In addition, since functions of the laser light beam cartridge and the cooling cartridge in the portable hair removal apparatus can be selected and controlled as necessary, the portable hair removal apparatus can be used as a laser light beam emitting apparatus which is used for various skin beauty treatments such as skin massage, skin whitening, and cuticle removal as well as hair removal. That is, the portable hair removal apparatus is versatile and has a high degree of usefulness.

Since the mounting portion in the body of the portable hair removal apparatus is equipped with a cooling fan, not only heat absorption and heat dissipation are performed through direct contact between the cooling cartridge and the skin, but also forceful cooling that blows air into the contact-type heat absorbing portion is used, the cooling effect thereof is improved. Furthermore, since heat dissipation is performed using the cooling fan even while the laser light beam cartridge is being used, it is possible to reduce skin irradiation even when the hair removal apparatus is used for a long period of time.

In addition, since the laser light beam outputting portion of the laser light beam cartridge is equipped with a touch sensor, a user can control the portable hair removal apparatus according to the user's intension by pressing buttons. For this reason, a user can use the portable hair removal apparatus for various skin beauty treatments. Furthermore, it is possible to prevent a laser light beam from being erroneously emitted when the portable hair removal apparatus is not in contact with skin. That is, it is possible to prevent accidents due to unintentional operation of the apparatus.

Since in the portable hair removal apparatus according to the present invention, the mounting portion of the body is equipped with an air circulation means that ventilates the mounting portion using outside air, the cooling effect thereof can be improved through air circulation performed by the air circulation means and via air blown by the cooling fan.

### BRIEF DESCRIPTION OF THE DRAWINGS

The above and other objects, features and other advantages of the present invention will be more clearly understood from the following detailed description when taken in conjunction with the accompanying drawings, in which:
FIG. 1 and 2 are perspective views illustrating a portable hair removal apparatus according to an embodiment of the present invention, in which the portable hair removal apparatus has a body to which respective cartridges are mounted in a replaceable manner;
FIGS. 3 and 4 are perspective views illustrating the portable hair removal apparatus from which the mounted cartridge is removed; and
FIGS. 5 and 6 are cross-sectional views illustrating the portable hair removal apparatus in which the cartridge is installed.

### DETAILED DESCRIPTION OF THE INVENTION

Reference will now be made in detail to various embodiments of the present invention, specific examples of which are illustrated in the accompanying drawings and described below, since the embodiments of the present invention can be variously modified in many different forms.

While the present invention will be described in conjunction with exemplary embodiments thereof, it is to be understood that the present description is not intended to limit the present invention to those exemplary embodiments. On the contrary, the present invention is intended to cover not only the exemplary embodiments, but also various alternatives, modifications, equivalents and other embodiments that may be included within the spirit and scope of the present invention as defined by the appended claims.

Throughout the drawings, the same reference numerals will represent members that have the same or like functions particularly when numbers in tenth digit and unit's digit are like or when numbers in tenth digit and unit's digit and alphabetical letters are like. Unless specifically set forth otherwise, all members designated by reference numerals are subject to this rule.

Each element and its shape may be schematically or exaggeratedly illustrated to help understanding of the invention. Some elements provided for a real product may not be illustrated or may be omitted in the drawings or description. The drawings should be construed only to aid understanding of the invention and should not be construed to limit the protection scope of the invention.

The terminology used herein is for the purpose of describing particular aspects or embodiments only and is not intended to be limiting. As used herein, the singular forms "a," "an" and "the" are intended to include the plural forms as well, unless the context clearly indicates otherwise.

It will be further understood that the terms "comprise", "include", "have", etc. when used in this specification, specify the presence of stated features, integers, steps, operations, elements, components, and/or combinations of them but do not preclude the presence or addition of one or more other features, integers, steps, operations, elements, components, and/or combinations thereof.

Unless otherwise defined, all terms including technical and scientific terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

It will be understood that, although the terms "first," "second," etc. may be used herein to describe various elements, these elements should not be limited by these terms. These terms are only used to distinguish one element from another element. For instance, a first element discussed below could be termed a second element without departing from the teachings of the present invention. Similarly, the second element could also be termed the first element.

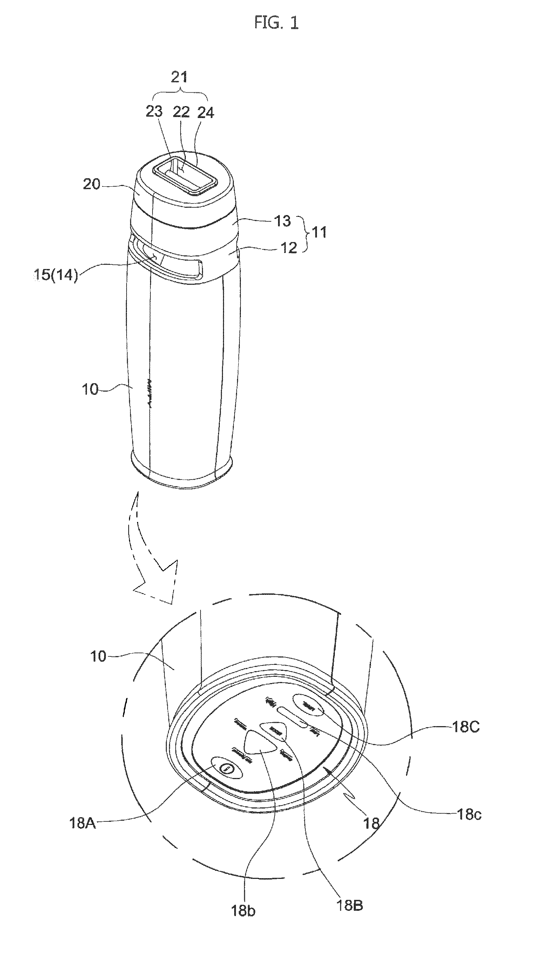

The definition of up and down herein uses gravity as a frame of reference in describing embodiments of the present invention. In reference to FIGS. 1 to 6, the direction of gravity is defined as down, the opposition direction of gravity is defined as up, and left-hand and right-hand sides are defined as left and right.

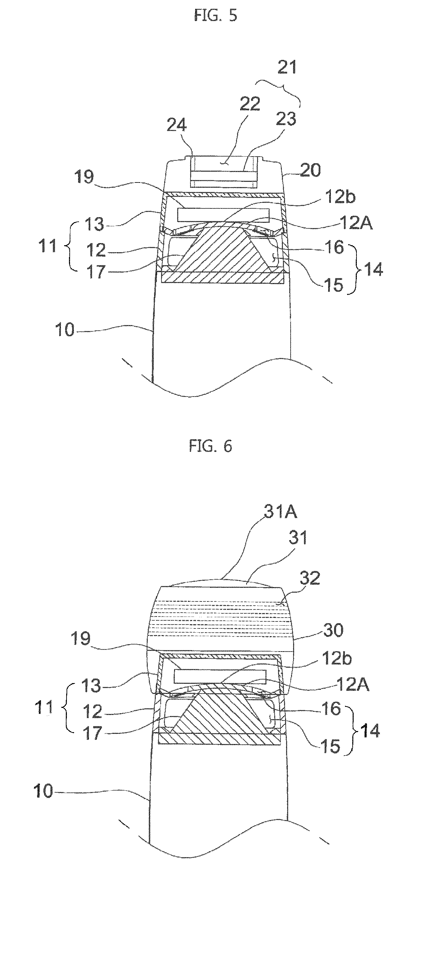

In FIG. 6, the side where either a cartridge 20 or a cartridge 30 is mounted is defined as front, the side where an operation button portion 18 disposed on a lower surface of a body 10 is mounted is defined as back. Accordingly, the terms "front" and "up" are interchangeably used and the terms "back" and "down" are interchangeably used herein during description. Furthermore, unless specifically set forth otherwise, directions in all the drawings and claims are described based on this rule.

The term "hair removal apparatus" or "laser light beam emitting apparatus" refers to all kinds of devices that emit laser light beams for the purpose of skin care therapy including hair removal. The terms "hair removal apparatus" and "laser light beam emitting apparatus" refer to the same device herein.

Hereinafter, a portable hair removal apparatus with a replaceable laser light beam cartridge and a replaceable cooling cartridge according to one embodiment of the present invention will be described with reference to the accompanying drawings.

With reference to FIGS. 1 to 6, a hair removal apparatus according to one embodiment of the present invention includes a laser light beam cartridge 20, a cooling cartridge 30, and a body 10 within which the laser light beam cartridge 20 are the cooling cartridge 30 can be selectively mounted.

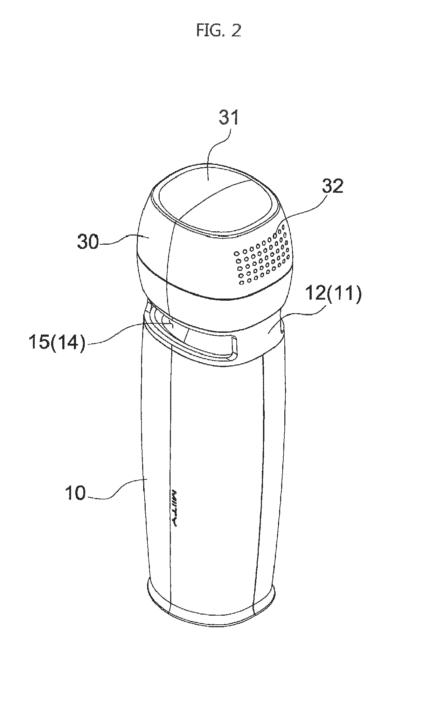

FIGS. 1, 3, and 5 illustrate a state in which the laser light beam cartridge 20 is removed from the body 10, and FIGS. 2, 4, and 6 illustrate the state in which the cooling cartridge 10 is removed from the body 10.

In FIGS. 5 and 6, the cross sections of only some parts are hatched but those of the others are not hatched for the purpose of simplifying illustration.

As illustrated in FIGS. 1 to 4, the body 10 has a streamlined form so that a user can easily hold it in user's hand. A controller (not shown) for controlling the cartridges 20 and 30 is built into the body 10.

As illustrated within a circle depicted in a lower portion of FIG. 1, the body 10 has a button portion 18 which allows a user to operate the cartridges 20 and 30. The button portion 18 is preferably disposed in the back surface (the bottom surface) of the body 10 so that there is no chance that a user unintentionally presses an operation button while using the hair removal apparatus.

The button portion 18 has a power button 18A for turning on and off the hair removal apparatus, a mode selection button 18B for mode switching between mode for the laser light beam cartridge 20 and mode for the cooling cartridge 30, and an adjustment button 18C for adjusting the function of each cartridge.

The mode selection button 18B is used to select an appropriate mode for a cartridge that is mounted within the body 10 after cartridge replacement is performed so that the replaced cartridge can properly operate.

That is, when hair removal mode is selected after the laser light beam cartridge 20 is mounted within the body 10, the body 10 will perform hair removal function. Meanwhile, when cooling mode is selected after the cooling cartridge 30 is mounted within the body 10, the body 10 will perform cooling function. When skin whitening mode is selected using the mode selection button 10B after the laser light beam cartridge 20 is mounted, the controller adjusts an output of a laser light beam that is emitted from the laser light beam outputting portion 21 so that the body 10 will function to remove cuticle, whiten skin, or give massage therapy effect to skin.

The adjustment button 18C is used to adjust an operation degree for each function. That is, the adjustment button 18C adjusts the intensity of the laser light beam in hair removal mode or the degree of cooling in cooling mode.

As for the adjustment of the output of the laser light beam, when the adjustment button 18C is pressed, a laser light beam having an intended frequency band is emitted and some effects can be obtained according to the frequency band of the laser light beam emitted. That is, when the adjustment button 18C is operated such that a laser light beam having a high frequency is output, an effect of hair root removal as well as downy hair removal and cuticle removal is obtained. On the other hand, when the adjustment button 18C is operated such that a laser light beam having a low frequency is output, an effect of removal of pimples and freckles is obtained through skin massage as well as an effect of skin whitening is obtained.

When the cooling cartridge 30 is mounted within the body 10, the adjustment button 18C can be operated to adjust the cooling temperature so that the hair removal apparatus according to the present invention can be used for skin care therapy.

The button portion 18 may further include a first display 18b that displays the selected mode according to operation of the mode selection button 18B and a second display 18C that displays the operation degree according to operation of the adjustment button 18C.

Accordingly, the hair removal apparatus according to the present invention can function as a multipurpose light emitting apparatus that gives various beauty treatment effects via irradiation of the skin via a laser light beam as well as a simple hair removal apparatus. That is, the hair removal apparatus according to the present invention has various functions.

The body 10 is equipped with a power supply means. Preferably, the power supply means may be a rechargeable battery (not shown) so as not to impair portability of the hair removal apparatus.

The body 10 has a USB terminal (not shown) that is connected to a rechargeable battery mounted within the body 10, so that a user can use the hair removal apparatus while charging the rechargeable battery by plugging in an electrical plug connected to the USB terminal.

As illustrated in FIGS. 1 to 6, the front side of the body 10 is provided with a mounting portion 11 to and from which the replaceable cartridge is mounted and / or removed.

The mounting portion 11 and the body 10 may be unified. Alternatively, the mounting portion 11 includes a first mounting member 12 and a second mounting member 13 in order to secure manufacturability of an air circulation means 14 described below. The first mounting member 12 is coupled to the front side of the body 10 and has a first corresponding coupling portion 12a to which a first coupling portion of the cooling cartridge 30 is coupled. The second mounting member 13 is coupled to the front side of the first mounting member 12 and has a second corresponding coupling portion 13a to which a second coupling portion 20a of the laser light beam cartridge 20 is coupled.

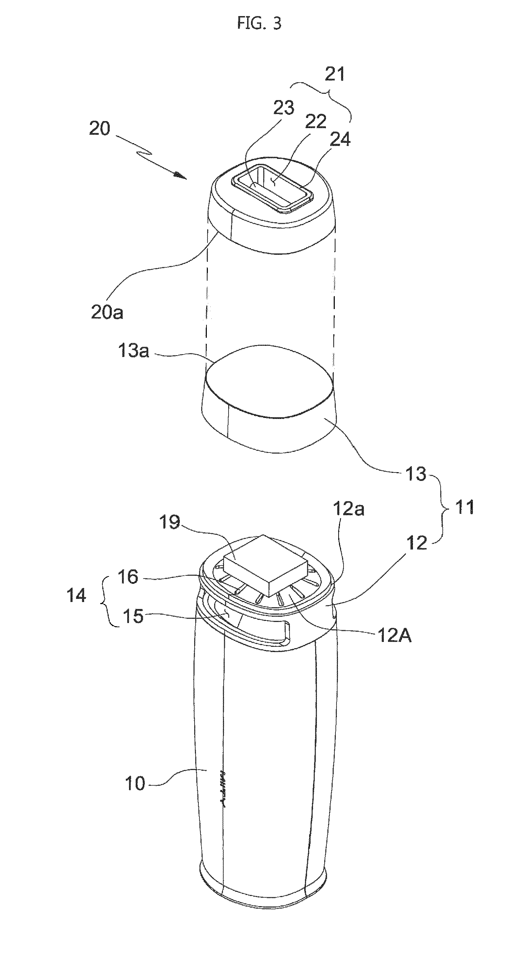

As illustrated in FIGS. 1 and 3, the laser light beam cartridge 20 is a cylindrical member which has the second coupling portion 20a disposed along the circumferential edge of a lower end portion thereof. The laser light beam cartridge 20 has the laser light beam outputting portion 21 in a front surface thereof.

The second coupling portion 20a of the laser light beam cartridge 20 and the second corresponding coupling portion 13a of the mounting portion 11 are known coupling members, i.e. male and female coupling members like a recess and a protrusion. The laser light beam cartridge 20 may be attached to and detached from the mounting portion 11 of the body 10 through a one-touch pressing attachment/detachment method which attaches or detaches the laser light beam cartridge 20 by pressing the laser light beam cartridge 20 against the mounting portion 11 of the body 10, a rotating attachment/detachment method that attaches or detaches the laser light beam cartridge 20 by rotating the laser light beam cartridge 20, or a buttoning attachment/detachment method that uses an additional button for attachment or detachment. The cooling cartridge 30 may also be attached to or detached from the body 10 using any one of the same attachment/detachment methods.

The laser light beam outputting portion 21 of the laser light beam cartridge 20 has a mounting recess 22 which is recessed from the front side to the back side. A light lamp 23 is installed in the mounting recess 22, is electrically connected to the power supply means and the controller, and emits a laser light beam.

The laser light beam output from the light lamp 23 is defined as intangible energy used for various beauty treatments including hair removal and skin massage using light. Representatives of the laser light beam include ultrasonic waves, laser, Intensive Pulse Light (IPL). Hereinafter, description is made about a laser light beam used for hair removal for simplicity of description.

A hair removal method using ultrasonic waves removes roots of body hairs by irradiating the skin with ultrasonic waves and is used for surgical operations or therapy. A hair removal method using laser removes roots of body hairs using light having a specific wavelength that transmits through a specific medium. A hair removal method using IPL removes roots of body hairs by emitting light with a predetermined wavelength in the form of periodic pulses.

When the hair removal methods using various forms of light are performed, light is absorbed by melanin in the skin and the absorbed light destroys hair roots. In this case, when the skin is irradiated with laser light for a long period of time, the exposed skin is irritated and damaged.

Therefore, appropriate post treatment should be performed at the same time as or after hair removal in order to cool and calm down the skin to ease the irritation of the skin. The present invention solves this problem by enabling the cooling cartridge 30, which enables the hair removal apparatus to cool down the skin by absorbing heat by coming into direct contact with the skin, to be mounted to the body 10 in place of the laser light beam cartridge 20.

To this end, as illustrated in FIGS. 2, 4, and 6, the cooling cartridge 30 takes the form of a cylindrical member having a first coupling portion 30a disposed along the circumferential edge thereof and is equipped with a contact-type heat absorbing portion 31 which appears when viewed from the front side. Therefore, when a user brings the contact-type heat absorbing portion 31 into contact with the skin from which body hair is removed, the contact-type heat absorbing portion 31 absorbs heat and transfers the heat to a rear portion of the cooling cartridge 30. In this way, the heat is eliminated.

Preferably, the contact-type heat absorbing portion 31 is made up of a member having high thermal efficiency. According to one embodiment of the present invention, the contact-type heat absorbing portion 31 is made of a thermoelectric element so that heat is dissipated through heat absorption and transfer. The skin is cooled down through this mechanism.

A representative of the thermoelectric element is a Peltier element which uses Peltier effect in which, when electric current is supplied to two different metals whose respective ends are in contact with each other, one metal absorbs heat and the other metal emits heat. Such a Peltier element is used in many industrial equipment and machines such as a cooling fan and a freezer.

In summary, as the exposed portion of the thermoelectric element that has high thermal efficiency, such as a Peltier element, functions as the contact-type heat absorbing portion 31, and the opposite portion, i.e., non-exposed portion connected to an inside portion of the cooling cartridge 30 functions as a heat sinking portion. The contact-type heat absorbing portion 31 absorbs heat as soon as it comes into contact with the skin, and the absorbed heat is dissipated through the back portion of the hair removal apparatus. In this way, skin calming effect and skin cooling effect are obtained.

Preferably, the contact-type heat absorbing portion 31 has as large contact area with respect to the skin as possible to enhance the heat dissipation effect and the cooling effect. Therefore, as illustrated in FIG. 6, the contact-type heat absorbing portion 31 has a protrusion 31A which protrudes from the front surface of a center portion thereof. The protrusion 31A has a gentle slope so that it is smoothly in contact with the skin.

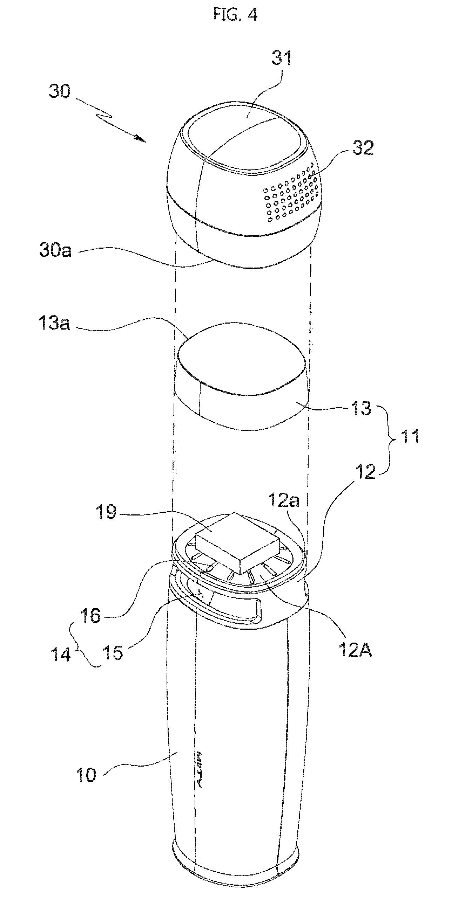

In order to increase the contact area of the contact-type heat absorbing portion 31 and enhance the heat dissipation effect using the cooling fan 19 and the air circulation means 14, the cooling cartridge 30 may take the form of a tube whose middle portion bulges as illustrated in FIG. 6, unlike the laser light beam cartridge 20 of FIG. 5. Therefore, the cartridge and the body 10 are assembled or disassembled in a manner that the first coupling portion 30a disposed at the lower end of the cooling cartridge 30 is not coupled to the second corresponding coupling portion 13a of the mounting portion 11 but is coupled to the first corresponding coupling portion 12a (as for the coupling portions, refer to FIGS. 3 and 4).

According to the invention, the thermoelectric element which forms the contact-type heat absorbing portion 312 of the cooling cartridge 30 is arranged to extend from the front side to the back side of the cooling cartridge. In this case, a back end portion of the thermoelectric element comes into contact with the upper surface of the second mounting member 13 so that absorbed heat can be dissipated. Accordingly, the cooling cartridge 30 is mounted to cover the second mounting member 13 so that the heat dissipating portion does not appear when viewed from outside. In this case, the first coupling portion 30a of the cooling cartridge 30 is coupled to the first corresponding coupling portion 13a of the first mounting member 12.

Such a structure of the cooling cartridge 30 allows the thermoelectric element which is built therein to have a maximum diameter, maximizing the heat absorption and dissipation effects.

In addition, as illustrated in FIGS. 4 and 6, the cooling cartridge 30 has a plurality of air holes 32 in the external surface thereof. Accordingly, air can be circulated around the thermoelectric element so that the heat dissipation effect can be enhanced.

Although not illustrated in the drawings, when either the laser light beam cartridge 20 or the cooling cartridge 30 is mounted to the mounting portion 11 of the body 10, when power is supplied to the laser light beam cartridge 20 or the cooling cartridge 30 via the coupling portion or an additional power supply means and when the controller controls the function of the laser light beam outputting portion 21 or the contact-type heat absorbing portion (thermoelectric element) according to operation of the button portion 18, the function of the portable hair removal apparatus is performed according to the selected mode and operation degree.

In addition, as illustrated in FIGS. 3 to 6, according to the present invention, the mounting portion 11 is equipped with the cooling fan 19 in order to cool down the laser light beam cartridge 20 or the cooling cartridge 30 as necessary.

Although the cooling fan 19 is illustrated to take the form of a box for the sake of convenient description, the cooling fan 19 may take any form as long as it can blow air into the second mounting member 13, with which the laser light beam cartridge 20 or the cooling cartridge 30 is in contact, to cool down the mounted cartridge 20 or 30.

The cooling fan 19 may be a well-known cooling member like a propeller-type cooling member which blows air using a propeller. The cooling fan 19 may be any type that can dissipate heat generated by each cartridge or by any part within the body 10 by blowing air.

The cooling fan 19 is preferably installed on an upper surface of a barrier slab 12A of the first mounting member 12 of the mounting portion 11. In this case, the second mounting member 13 functions as housing for covering the cooling fan 19 as well as functions as the mounting portion 11 of the laser light beam cartridge 20.

Although not illustrated, the cooling fan 19 operates on power supplied from a rechargeable battery installed in the body 10 and it is connected to the controller. The cooling fan 19 may be interlocked with each cartridge so that the cooling fan 19 may start operating at the same time as the cartridge operates. Alternatively, the cooling fan 19 may operate when a button for operation of the cooling fan 19 is pressed.

In addition, the hair removal apparatus according to the present invention has a heat-dissipating design structure for ventilating the mounting portion 11 in order to enhance the cooling effect of the cooling fan 19.

That is, as illustrated in FIGS. 3 to 6, the mounting portion 11 is further equipped with the air circulation means 14 for ventilating the mounting portion with outside air.

The air circulation means 14 has a plurality of air circulation holes 16 formed to penetrate through the barrier slab 12A of the first mounting member 12.

The air circulation holes 16 are formed by boring holes in the barrier slab 12A, along the edge. The air circulation holes 16 are arranged in a radial pattern.

The first mounting member 12 and the second mounting member 13 communicate with each other through the air circulation holes 16 so that air can circulate through the inside of the mounting members, promoting heat dissipation from the cartridges.

Furthermore, since the cooling fan 19 is installed on the barrier slab 12A of the first mounting member 12, the barrier slab 12A has a bulging portion 12b (see FIG. 5) which protrudes from an upper surface in the center portion thereof. The cooling fan 19 is arranged in place on the bulging portion 12a. In this case, a space is created between the barrier slab 12A and the cooling fan 19, promoting air ventilation.

In addition, as illustrated in FIGS. 3 to 6, the air circulation means 14 includes a plurality of air circulation holes 15 formed to penetrate through the exterior surface of the first mounting member 12.

The air circulation holes 15 may take the form of a slit like the air circulation hole 16 and may be arranged along the edge portion of the first mounting member 12. In order to maximize air circulation effect, the air circulation holes 15 having a rectangular shape are arranged along the circumferential edge of the first mounting member as illustrated in the drawings.

When the cooling fan 19 starts operating, air inside the first mounting member 12 flows in through the air circulation holes 16 and blows upward. With this air circulating structure, outside air is introduced through the air circulation holes 15 and inside air with room temperature is continuously blown into the inside of the mounting portion 11. In this way, cooling is performed.

Each of the cartridges may have a plurality of auxiliary circulation holes (not shown) which is formed to penetrate through the corresponding cartridge in a vertical direction and which communicates with the air holes 32. This structure also enhances the cooling effect.

In addition, as illustrated in FIGS. 5 and 6, the mounting portion 11 has an inclined guide portion 17 which slops up from the edge of the bottom of the first mounting member 12 toward the center of the first mounting member 12. The inclined guide portion 17 causes outside air, which flows in through the air circulation holes 15, to smoothly travel to the cooling fan 19.

That is, the inclined guide portion 17 is a conical member formed within the first mounting member 12. The guide portion 17 tapers to the upper center portion thereof so that the side wall of the inclined guide portion 17 is sloped up toward the center. Therefore, the air, which is blown by the cooling fan 19, travels up along the inclined wall of the inclined guide portion 17 when it comes into contact with the inclined guide portion 17 so that the air is efficiently introduced into the second mounting member 13.

The inclined guide portion 17 may be made of an element that can effectively transfer heat, for example, a thermoelectric element like the contact-type heat absorbing portion of the cooling cartridge in order to improve cooling effect as well as it functions to guide outside air.

Accordingly, the hair removal apparatus according to the present invention has the effect of efficiently dissipating heat generated within the hair removal apparatus and the effect of maximizing the cooling performance of the cooling cartridge 30 by continuously cooling the thermoelectric element (i.e. contact-type heat absorbing portion 31) of the cooling cartridge 30.

Since the hair removal apparatus according to the present invention has a heat dissipating structure such as the cooling fan 19 and the air circulation means 14 which maximize the effect of cooling and dissipating heat from the mounting portion 11 of the body 10 and each cartridge, heat trapped in the skin is reliably absorbed and dissipated and the skin is effectively cooled down. That is, the present invention improves the skin calming effect as well as the basic function (i.e. hair removal function) of the laser light beam cartridge. Furthermore, the hair removal apparatus may function as a multi-purpose light emitting apparatus that is used for diverse skin beauty treatments such as skin whitening, skin massaging (especially, cold massaging), and cuticle removal.

In addition, as illustrated in FIGS, 1 and 3, the laser light beam cartridge 21 of the laser light beam cartridge 20 is equipped with a touch sensor 24.

The touch sensor 24 is mounted around the edge of the mounting recess 22 to improve manufacturability of the hair removal apparatus. When the laser light beam cartridge 20 is mounted in the body of the hair removal apparatus and when a user brings the laser light beam outputting portion 21 into contact with the skin, the touch sensor 24 detects the skin and a laser light beam is emitted from the light lamp 23.

The touch sensor 24 may be a simple touch sensing type or a luminance sensing type. Alternatively and preferably, it may be a capacitive sensing type in order to prevent the touch sensor 24 erroneously operating when the laser light beam cartridge 20 comes into contact with an object other than the skin.

The touch sensor 24 operates by interlocking with the controller built in the body, thereby controlling outputting of a laser light beam from the light lamp 23 when it senses the touching of the laser light beam cartridge to the skin.

Controlling the outputting of a laser light beam using the touch sensor 24 is executed in various ways. For example, there are methods including a method of outputting a laser light beam when it takes a predetermined period of time after the touching of the laser light beam cartridge to the skin is sensed, a method of outputting a laser light beam when a predetermined count of touches are sensed, a method of periodically outputting a laser light beam when the touching is continuously sensed after a first laser light beam is output through operation of a laser light beam outputting button, and a method of controlling the count of outputs of a laser light beam when touching is sensed.

Although not illustrated in the drawings, the laser light beam cartridge 20 may be configured such that the laser light beam outputting portion 21 is equipped with a light filter so that it primarily selects a wavelength of a laser light beam to be emitted using the filter.

The filter is installed to close an upper opening of the mounting recess 22 and is made of a light transmittable material so that a wavelength of a laser light beam to be emitted is automatically selected as light passes through the filter.

This is because laser light beams having same frequency may have different impacts on different types of skin (bodies) according to status, condition, and constitution of the skin. When only the intensity of the laser light beam is adjusted using the adjustment button 18C, there may be unintentional negative impact on a user's skin. However, when the filter is used, it is possible to prevent this accident.

To this end, the filter is detachably installed in the mounting recess 22. Furthermore, a manufacturer provides a user with a plurality of filters so that the user can obtain a laser light beam having an intended wavelength band by selectively using any of the plurality of filters according to the user's intention.

For convenience of description, the present invention is described referring to an embodiment that describes a portable hair removal apparatus as a representative example, it is apparent that the hair removal apparatus of the present invention can be used as a laser light beam emitting apparatus for various skin beauty treatments when the hair removal apparatus of the present invention is used as a hand piece or a probe connected to a separate body. That is, the description which has been made herein above should not be construed so as to limit or restrict the scope of the present invention.

Although the present invention has been described referring to an embodiment of a portable hair removal apparatus having a replacement laser light beam cartridge or a replacement cooling cartridge with reference to the accompanying drawings herein above, those skilled in the art will appreciate that various modifications, additions and substitutions are possible, and various alternatives, modifications, equivalents and other embodiments may be included within the spirit and scope of the present invention.

## Claims

1. A portable hair removal apparatus, comprising:
a main body with a mounting portion;
a laser light beam cartridge which is mounted to or removed from the mounting portion and which is equipped with a laser light beam outputting portion; and
a cooling cartridge which is mounted to and removed from the mounting portion and is equipped with a contact-type heat absorbing portion.

2. The portable hair removal apparatus according to claim 1, wherein the mounting portion is equipped with a cooling fan.

3. The portable hair removal apparatus according to claim 1 or 2, wherein the mounting portion is equipped with an air circulation portion through which external air is introduced.

4. The portable hair removal apparatus according to any of claims 1 to 3, wherein the laser light beam outputting portion is equipped with a touch sensor.
